# EUROPÄISCHE PATENTSCHRIFT

(11) **EP 3 784 769 B1**
(45) Veröffentlichungstag und Bekanntmachung des Hinweises auf die Patenterteilung: **10.11.2021**
(21) Anmeldenummer: 19790438.6
(22) Anmeldetag: 19.09.2019
(51) Int. Cl.: C12M 1/107, C12M 1/00, C12M 1/02, C12M 1/33

(54) **VORRICHTUNG UND VERFAHREN ZUR OPTIMIERUNG EINER BIOGASANLAGE**
DEVICE AND METHOD FOR OPTIMIZING A BIOGAS SYSTEM
DISPOSITIF ET PROCÉDÉ POUR OPTIMISER UNE INSTALLATION DE BIOGAZ

(30) Priorität: 19.09.2018 DE 102018123058
(43) Veröffentlichungstag der Anmeldung: 03.03.2021
(73) Patentinhaber: Maier, Clemens, 88316 Isny-Sommersbach (DE); Maier, Gregor, 88316 Isny-Sommersbach (DE)
(72) Erfinder: Maier, Clemens, 88316 Isny-Sommersbach (DE); Maier, Gregor, 88316 Isny-Sommersbach (DE)
(74) Vertreter: Höchtl, Walter
(86) Internationale Anmeldenummer: PCT/DE2019/100824
(87) Internationale Veröffentlichungsnummer: WO 2020/057697

(56) Entgegenhaltungen:
- WO-A1-2015/067240
- DE-A1- 4 114 667
- DE-A1-102012 024 108
- DE-T2- 60 218 132
- DE-U1-202007 017 698
- MELSA ARMIN ET AL: "Trocknung kommunaler Klaerschlaemme in DeutschlandTeil 2: Erfahrungen mit bestehenden Anlagen", KORRESPONDENZ ABWASSER, ABWASSERTECHNISCHE VEREINIGUNG, ST. AUGUSTIN, DE, Bd. 46, Nr. 9, 1. Januar 1999 (1999-01-01) , Seiten 1445-1456, XP002186816, ISSN: 0341-1540

## Beschreibung

Die Erfindung betrifft eine Vorrichtung und ein Verfahren zur Beschickung eines Fermenters.

Aus der deutschen Offenlegungsschrift DE 41 14 667 A1 ist ein Verfahren und eine Vorrichtung zur Aufbereitung von in Form von Gülle vorliegender tierischer Abfallprodukte bekannt, dass darin besteht, dass die Abfallprodukte zunächst in einem Fermenter zur Bildung von Methangas gelagert werden, von dort kontinuierlich einer Zentrifuge zugeführt werden und die zentrifugierte feste Komponente anschließend getrocknet wird. Die in der Zentrifuge abgetrennte flüssige Komponente wird in einen Verdampferkessel geleitet, in dem das darin enthaltene Wasser durch Unterdruck verdampft, anschließend in einem Kondensator wieder verflüssigt und dem natürlichen Wasserkreislauf zugeführt wird.

Aus dem deutschen Gebrauchsmuster DE 202007017698 U1 ist eine Fermentervorrichtung bekannt, die Fermentationsbrühen zu Biogas umsetzt, mittels einer Separationseinrichtung zur Auftrennung der Fermentationsbrühe in eine Dick- und in eine Dünnfraktion. Hier wird die Dünnfraktion keinem Verdampfer zugeführt, sondern direkt in einem Fermenter vergoren.

Die deutsche Patentanmeldung DE 102012024108 A1 offenbart ebenso eine Vorrichtung und ein Verfahren zum Betrieb einer Biogasanlage mittels Separator, der den Klärschlamm in eine Dick- und in eine Dünnphase trennt.

Die Dünnphase wird dem Kulturwasser von aquatischen Kulturpflanzen zugegeben.

Die Verfahrensweise im Umgang mit der Dünnphase bzgl. der beiden zuletzt genannten Schriften gilt es seitens der vorliegenden Anmeldung zu vermeiden, da dies wegen des hohen Volumenanteils an Flüssigkeit im Vergleich zum geringen Feststoffanteil unwirtschaftlich ist (Baukosten großer Behälter) und energetisch ineffizient, da große Wärmemengen zur Temperierung benötigt werden.

Aus der deutschen Patentschrift DE 602 18 132 T2 ist ein Verfahren und eine Anlage zu Konversion biologisch abbaubarer Materialien in ein Produktgas bekannt. Diabei wird das Ausgangsmaterial vor der Anaeroben Faulung durch Beimischung von Flüssigkeit für eine optimale Biogaserzeugung aufbereitet. Dies führt zu einer Erhöhung des Volumens des Ausgangsmaterials, so dass entsprechend große Faulbehälter benötigt werden.

Die zur Methangasproduktion in Fermentern vorgesehene Biomasse, die nicht nur, aber insbesondere bei Gülle aus einem hohen Flüssiganteil besteht, wird in der Regel in sogenannten Vorgruben bereitgestellt. Wird die aus Flüssigkeit und Faseranteil bestehende Biomasse, direkt in einen Fermenter geleitet, muss der Fermenter ein ausreichend großes Volumen aufweisen, was sich negativ auf die Bau-, Anlage- Lager und Betriebskosten auswirkt. - Relativ hohe Betriebskosten fallen beispielsweise auch für die Beheizung der zu fermentierenden Biomasse an. Aufgabe der Erfindung ist es daher Vorrichtungen und Verfahren zur Beschickung eines Fermenters einer Biogasanlage zur Verfügung zu stellen. Je nach Bedarf und Wirtschaftlichkeit kann eine erfindungsgemäße Vorrichtung hinsichtlich der Volumenreduktion oder hinsichtlich der Energieeffizienz (Wärmeenergie für den Fermenter) optimiert werden. Beiden hier beschriebenen Lösungen ist gemeinsam, dass die Biomasse unmittelbar nach der Vorgrube in Feststoff- (Faseranteile) Flüssiganteil (im Folgenden auch als Dünnschlamm bezeichnet), separiert wird.

Diese Aufgabe wird mit einer Vorrichtung gemäß den Ansprüchen 1 und 5 zur Beschickung eines Fermenters einer Biogasanlage durch Volumenreduktion von Biomasse gelöst, die folgende Merkmale umfasst:
einen mechanischen Separator, mit einem Einlass für die Biomasse, einen Feststoffauslass, und einen Dünnschlammauslass, einen Fermenter mit einem Feststoffeinlass, einem Dickschlammeinlass und einem Gärresteauslass,
eine Verdampfungsanlage mit einem Dünnschlammeinlass, einem Dickschlammauslass, einem Kondensatauslass und einem Auslass für anfallende Ammoniumsulfatlösung,
wobei der mechanische Separator über eine Pumpe und eine erste Leitung mit der Vorgrube, sein Feststoffauslass mit dem Feststoffeinlass des Fermenters und sein Dünnschlammauslass mit dem Dünnschlammeinlass der Verdampfungsanlage verbunden ist und wobei der Dickschlammauslass der Verdampfungsanlage auf den Dickschlammeinlass des Fermenters geführt ist.

Die Volumenreduktion der Biomasse direkt nach der Vorgrube hat mindestens folgende Vorteile. Die Anlagen für die Produktion von Biogas (Fermenter, Beschickungsanlagen, etc.) können kleiner gebaut werden oder bei gleicher Größe kann mehr produziert werden. Zweitens wird durch die Verdampfung in diesem "Vorstadium" die Biomasse erwärmt, so dass durch den erwärmten Dickschlamm eine Beheizung des Fermenters nicht mehr oder nur in geringerem Umfang erforderlich ist. Zusätzlich kann auch durch Wärmerückgewinnung aus dem oder den Verdampfern Wärme zur Beheizung des Fermenters gewonnen werden.

Eine vorteilhafte Ausbildung der Vorrichtung besteht darin, dass der Gärresteauslass des Fermenters über eine zweite Leitung und die Pumpe auf den Einlass für die Biomasse und über eine fünfte Leitung zu einem ersten Gärresteausgang geführt ist, wobei der Dickschlammauslass der Verdampfungsanlage über eine vierte Leitung auf einen zweiten Gärresteauslass geführt ist und wobei die erste bis fünfte Leitung jeweils mit einem ersten bis fünften Schieber versehen sind.

Durch die (wiederholte) Rückführung der fermentierten Biomasse vom Fermenter an den Anlageneingang kann die Biomasse weiter reduziert und der Wirkungsgrad der Biogasproduktion erhöht werden. Zusätzlich kann in der Verdampfungsanlage weiteres Ammonium entzogen werden.

Die Aufgabe wird durch ein Verfahren gemäß Anspruch 3 zur Optimierung einer Biogasanlage gelöst,
wobei in einer Vorgrube gelagerte Biomasse in einen mechanischen Separator gepumpt und dort in Feststoff und Dünnschlamm getrennt wird, wobei der Dünnschlamm in einer Verdampfungsanlage zu Dickschlamm eingedampft wird und
wobei der Feststoff sowie der Dickschlamm in einen Fermenter geleitet werden.

Eine vorteilhafte Ausbildung des Verfahrens besteht darin, dass die im Fermenter fermentierten Gärreste auf den Separator zurückgeführt werden und nach mindestens einem Durchlauf aus der Verdampfungsanlage über einen zweiten Gärresteauslass ausgeschleust werden.

Die Optimierung einer Biogasanlage hinsichtlich der Energieeffizienz, lässt sich mit den Gegenständen gemäß den Ansprüchen 5 und 6 lösen. Durch die Wärmerückgewinnung aus dem Flüssiganteil (Dünnschlamm) der vergorenen Biomasse, beispielsweise von Gülle, der mittels eines dem Fermenter nachgeordneten Separators gewonnen wird, lässt sich deutlich Heizenergie einsparen.

Beispielsweise muss die Biomasse im Fermenter von 10°C (die Temperaturangaben beziehen sich auf die durchschnittliche Wärme von Gülle über das Jahr gesehen) auf 40°C erwärmt werden. Die dafür bei herkömmlichen Anlagen verwendeten Gülle/Gülle Wärmetauscher - also Wärmetauscher durch deren Kreisläufe Biomasse inklusive der Faseranteile gepumpt wird - sind sehr kostenintensiv, da sie so ausgelegt werden müssen, dass sie mit den Feststofffasern der nicht vorher separierten Biomasse zurechtkommen müssen. Beispielsweise kosten Wärmetauscher, die in der Lage sind, täglich 100 m³ bis 120m³ Gülle zu erwärmen, Stand heute ca. 150.000€ und benötigen etwa den fünf fachen Platz im Vergleich zu einem Wärmetauscher, in dessen Kreisläufe jeweils nur der Flüssiganteil der Gülle gegeben wird. Letztere Wärmetauscher sind um den Faktor 10 günstiger. (Die Bezeichnung Gülle/Gülle Wärmetauscher steht hier allgemein für Wärmetauscher, die für flüssige Biomasse jeglicher Art vorgesehen sind.).

Hinsichtlich der Energieeffizienz wird die Aufgabe mit einer Vorrichtung und einem Verfahren zur Beschickung des Fermenters einer Biogasanlage durch Wärmerückgewinnung aus dem Dünnschlammanteil von vergorener Biomasse gelöst, wie in den Patentansprüchen 5 und 6 beschrieben. Diese Lösung schlägt demnach vor, den Flüssiganteil vor dem Fermenter zu separieren und über einen Wärmetauscher mit dem separierten Flüssiganteil der aus dem Fermenter gewonnenen Gärreste zu erwärmen und dann dem Fermenter zuzuführen.

Die Vorrichtungen und die Verfahren werden im Folgenden anhand der Figuren näher beschrieben. Dabei zeigen:
- Figur 1: eine schematische Darstellung einer Anlage zur Beschickung des Fermenters einer Biogasanlage hinsichtlich Volumenreduktion
- Figur 2: die Anlage gemäß Figur 1 mit Rückführung von fermentierter Biomasse an den Anlageneingang
- Figur 3: eine schematische Anlage zur Beschickung des Fermenters einer Biogasanlage hinsichtlich Energieeffizienz, mit Wärmerückgewinnung aus und für den Fermenter

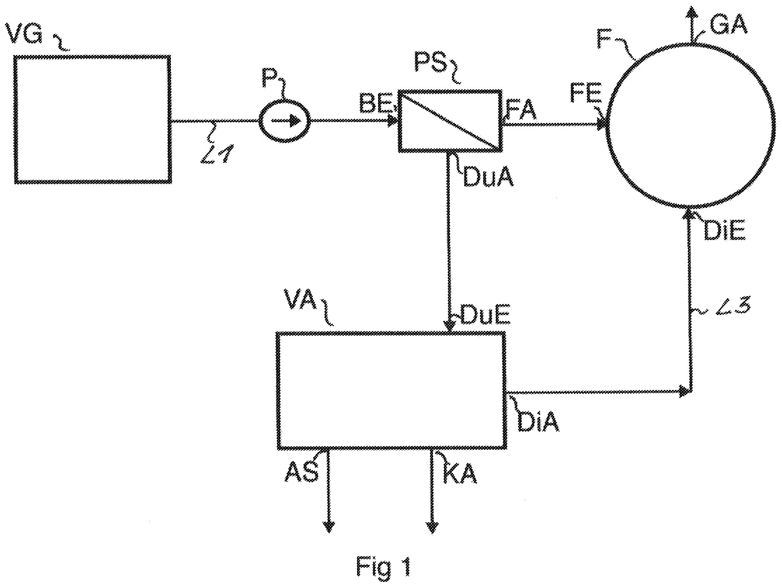

Die schematische Darstellung gemäß Figur 1 zeigt eine Vorrichtung zur Volumenreduktion von Biomasse. Diese Biomasse, wird in der Regel in einer sogenannten Vorgrube VG gesammelt. Von dort kann sie bei Bedarf über die Pumpe P und den Biomassen-Eingang BE in den mechanischen Separator PS gepumpt werden. Als Separatoren PS können mechanische Separatoren wie beispielsweise ein Pressschnecken-, ein Siebtrommel- oder ein Rollenseparator Verwendung finden.

Im Separator PS wird die Biomasse in Feststoff- und Dünnschlamm getrennt. Als Dünnschlamm wird der separierte Flüssigkeitsanteil der Biomasse bezeichnet, der je nach Größe des Siebes im Separator noch Feststoffpartikel enthält. Der Feststoff wird über den Feststoffausgang FA dem Fermenter F über dessen Feststoffeingang FE zugeführt. Der Dünnschlamm wird über den Dünnschlammausgang DuA des Separators der Verdampfungsanlage VA über deren Dünnschlammeingang DuE zugeführt.

Die Verdampfungsanlage ist hier nur als Black Box dargestellt. Derartige Verdampfungsanlagen sind beispielsweise in der Biogastechnik hinreichend bekannt. Beispielsweise ist in der älteren deutschen Patentanmeldung DE 10 2017 118 356 eine Verdampfungsanlage mit einem Wärmetauscher, einem Verdampfer, einem Brüdenwäscher, einem Kondensator, etc., bekannt. Aus dem im Verdampfer verdampften Brüden wird im Brüdenwäscher das Ammoniumsulfat als Ammoniumsulfatlösung ausgewaschen und der gereinigte Dampf wird kondensiert. In der hier beschriebenen Vorrichtung wird die Ammoniumsulfatlösung über den Ausgang AS und das anfallende Kondensat über den Ausgang KA aus der Verdampfungsanlage VA ausgeleitet. Die Verdampfungsanlage kann auch mehrstufige Verdampfer aufweisen, wie sie beispielsweise in der internationalen Anmeldung WO2015067240A1 beschrieben sind. Beheizt wird die Verdampfungsanlage in der Regel über ein hier nicht näher dargestelltes Blockheizkraftwerk, wobei die Verdampfungswärme - die bei mehreren Verdampfern am Ende der Kaskade noch zur Verfügung steht - zur Beheizung des Fermenters oder voriger Aufheizung der zu fermentierenden Biomasse benutzt werden kann. Je nach Wärme, die vom Blockheizkraftwerk geliefert wird und der Anzahl der Verdampferstufen, kann das Volumen der Biomasse und entsprechend die dafür nötigen Anlagenkomponenten gegenüber herkömmlichen Anlagen um 20% bis 60% reduziert werden.

Der in der Verdampfungsanlage VA eingedickte Dünnschlamm wird als Dickschlamm über den Dickschlammausgang DiA in den Fermenter F über dessen Dickschlammeingang DiE verbracht. Die im Fermenter F fermentierte Biomasse kann als Gärrest über den Gärresteausgang GA zum Beispiel in ein hier nicht dargestelltes Gärrestelager verbracht werden. Durch die Verdampfung und die Reduzierung des Volumens der Biomasse muss einerseits weniger Flüssigkeit verbracht werden, andrerseits kann auch das Gärrestelager kleiner ausgeführt werden.

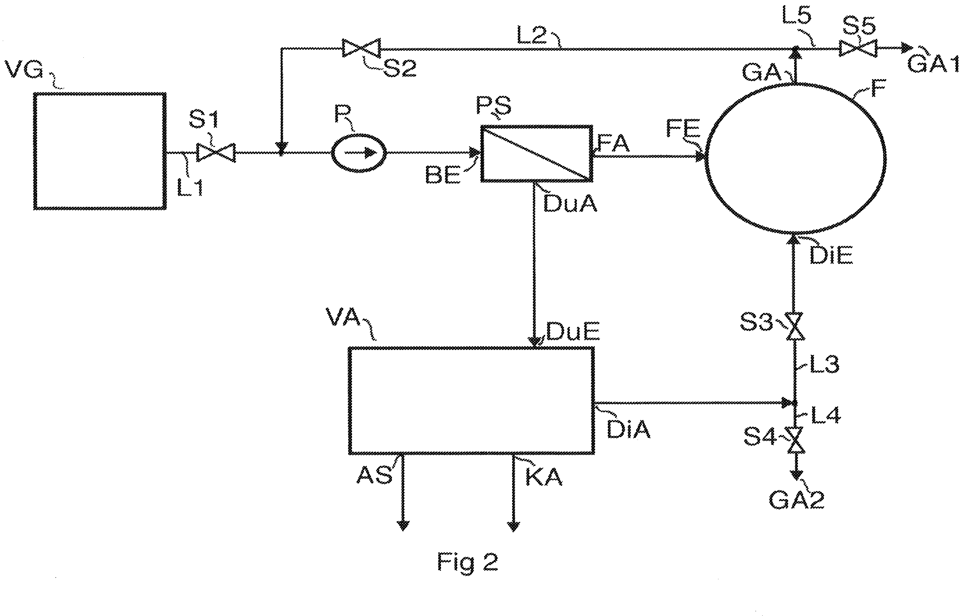

In der Darstellung gemäß Figur 2 ist der Separator PS über die erste Leitung L1, einen ersten Schieber S1 und über die Pumpe P mit der Vorgrube VG verbunden. Nach dem ersten Schieber S1 ist eine zweite Leitung L2 vom Gärresteausgang GA des Fermenters F über einen zweiten Schieber S2 an die erste Leitung L1 geführt. Ein dritter Schieber S3 ist in der dritten Leitung L3, die zum Fermenter F führt, angebracht und ein vierter Schieber S4 befindet sich in der Leitung S4, die mit dem Dickschlammausgang DiA der Verdampfungsanlage VA verbunden ist. Die vierte Leitung L4 führt zu einem zweiten Gärresteausgang GA2. Eine fünfte Leitung L5 führt vom Gärresteausgang GA über einen fünften Schieber S5 zum ersten Gärresteausgang GA1.

Werden der erste, der dritte und der fünfte Schieber S1, S3, S5 geschlossen und der zweite und der vierte Schieber S2, S4 geöffnet, kann die im Fermenter F fermentierte Biomasse über den Gärresteausgang GA, mittels der Pumpe P in den Separator PS gepumpt werden und dort nochmals in Feststoff und Dünnschlamm getrennt werden.

Der Feststoff wird zur weiteren Fermentierung in den Fermenter F verbracht. Der in der Verdampfungsanlage VA eingedickte Dünnschlamm kann schließlich als Dickschlamm über den Dickschlammausgang DiA, die vierte Leitung L4 und den offenen, vierten Schieber S4 und den zweiten Gärresteausgang GA2 ausgebracht werden.

Werden der erste und der dritte Schieber S1, S3, geöffnet und der zweite, der vierte und der fünfte Schieber S2, S4, S5 geschlossen, funktioniert die Anlage wie anhand der Figur 1 beschrieben. Die fermentierten Gärreste können dann durch Öffnen des Schiebers S5 aus dem Fermenter F entnommen werden.

Auf die Beschreibung und Darstellung von im Anlagenbau bekannten Elementen die dem Fachmann geläufig sind und die er je nach Anlagenkonstruktion vorsehen wird, wie beispielsweise weitere Pumpen, ist der Übersichtlichkeit und klaren Darstellung wegen verzichtet worden. Nur beispielhaft sei darauf hingewiesen, dass der Feststoffeintrag in den Fermenter auch über bekannte Eintragsvorrichtungen, wie Förderschnecken, Förderbänder, Pumpen, etc., erfolgen kann.

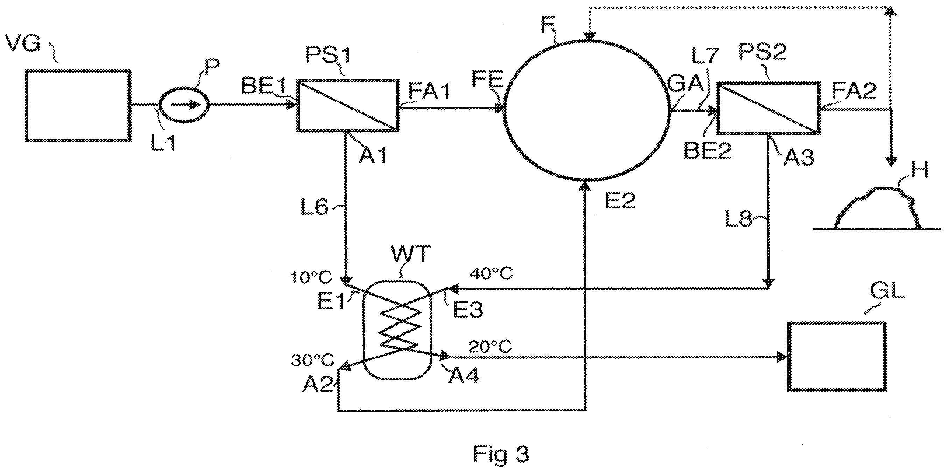

Die Darstellung gemäß Figur 3 zeigt eine schematische Anlage zur Beschickung des Fermenters einer Biogasanlage hinsichtlich Energieeffizienz, mit Wärmerückgewinnung aus und für den Fermenter. Dabei wird die Wärme der Flüssiggärreste aus dem Fermenter F genutzt um den vorher aus dem ersten Separator gewonnenen Flüssiganteil (Dünnschlamm) auf eine höhere Temperatur zu bringen, bevor dieser Flüssiganteil dem Fermenter zugeführt wird. Dazu ist an den Ausgang A1 des bereits bekannten mechanischen ersten Separators PS1 ein erster Kreislauf eines Wärmetauschers WT mit dem Eingang E1 und dem Ausgang A2 angeschlossen, wobei der Ausgang A2 des ersten Kreislaufs in den Fermenter geführt ist. Der Flüssiganteil des ersten Separators PS1 hat beispielsweise eine Temperatur von ca. 10°C.

Der Feststoffanteil aus dem ersten Separator PS wird wie oben bereits beschrieben, in den Fermenter F gespeist. Über den Gärresteausgang GA des Fermenters F werden die Gärreste (ca. 40°C) in den zweiten Separator PS2 gegeben und dort wieder in Feststoff und Flüssiganteil getrennt.

Der Feststoffanteil kann auf eine Halde zur weiteren Verbringung gegeben werden, oder alternativ in den Fermenter zurückgeführt werden (gestrichelte Leitung). Nach mehreren Durchläufen und abhängig von der Biomasse, insbesondere bei Gülle, sind der Feststoffanteil, d. h. dessen Partikel, so klein, dass sie im Flüssiganteil mitschwimmen, d. h., dass kein eigentlicher Feststoffanteil mehr separiert wird, weil die Feststoffpartikel vom Sieb des Separators nicht mehr zurückgehalten werden.

Der Flüssiganteil aus dem zweiten Separator PS2, der aus den ca. 40°C warmen Gärresten separiert wurde, wird dem zweiten Kreislauf des Wärmetauschers WT über den dritten Eingang E3 zugeführt und gibt seine Wärme an den ersten Kreislauf ab, bevor er über den Ausgang A4 in das Gärrestelager GL verbracht wird.

Über den Ausgang A2 des Wärmetauschers WT wird somit der (hier beispielsweise um 20°C) erwärmte Flüssiganteil in den Fermenter F gegeben, so dass die Energie für dessen Beheizung deutlich reduziert werden kann.

Die Gülle, die Gärreste und der jeweilige Dünnschlamm werden mittels Pumpen transportiert. So sind neben der Pumpe P noch mindestens je eine Pumpe in den Leitungen L6, L7 und L8 vorgesehen.

Die angegebenen Temperaturen sind zur Verdeutlichung angegeben. In der Praxis können diese natürlich abweichen. Bei dem Wärmetauscher handelt es sich um einen Gegenstromwärmetauscher.

## Patentansprüche

1. Vorrichtung zur Beschickung eines Fermenters einer Biogasanlage durch Volumenreduktion von in einer Vorgrube (VG) gelagerter Biomasse umfassend, einen mechanischen Separator (PS), mit einem Einlass (BE) für die Biomasse, einen Feststoffauslass (FA), und einen Dünnschlammauslass (DuA),
einen Fermenter (F) mit einem Feststoffeinlass (FE), einen Dickschlammeinlass (DiE) und einem Gärresteauslass (GA),
eine Verdampfungsanlage (VA) mit einem Dünnschlammeinlass (DuE), einem Dickschlammauslass (DiA), einem Kondensatauslass (KA) und einem Auslass (AS) für anfallende Ammoniumsulfatlösung,
wobei der mechanische Separator (PS) über eine erste Leitung (L1) mit der Vorgrube (VG), sein Feststoffauslass (FA) mit dem Feststoffeinlass (FE) des Fermenters (F) und sein Dünnschlammauslass (DuA) mit dem Dünnschlammeinlass (DuE) der Verdampfungsanlage (VA) verbunden ist und wobei der Dickschlammauslass (DiA) der Verdampfungsanlage (VA) auf den Dickschlammeinlass (DiE) des Fermenters (F) geführt ist.

2. Vorrichtung nach Anspruch 1, wobei der Gärresteauslass (GA) des Fermenters (F) über eine zweite Leitung (L2) auf den Einlass (BE) für die Biomasse und über eine Leitung L5 zu einem ersten Gärresteausgang (GA1) geführt ist,
wobei der Dickschlammauslass (DiA) der Verdampfungsanlage (VA) über eine vierte Leitung (L4) auf einen zweiten Gärresteauslass (GA2) geführt ist und wobei die erste bis fünfte Leitung (L1 bis L5) jeweils mit einem ersten bis fünften Schieber (S1 bis S5) versehen sind.

3. Verfahren zur Beschickung eines Fermenters einer Biogasanlage durch Volumenreduktion von in einer Vorgrube (VG) gelagerter Biomasse, wobei die Biomasse aus der Vorgrube (VG) in einen mechanischen Separator (PS) gepumpt und dort in Feststoff und Dünnschlamm getrennt wird, wobei der Dünnschlamm in einer Verdampfungsanlage (VA) zu Dickschlamm eingedampft wird und wobei der Feststoff sowie der Dickschlamm in einen Fermenter (F) geleitet werden.

4. Verfahren nach Anspruch 3, wobei die im Fermenter (F) fermentierten Gärreste auf den Separator (PS) zurückgeführt werden und nach mindestens einem Durchlauf aus der Verdampfungsanlage (VA) über einen zweiten Gärresteauslass (GA2) ausgeschleust werden.

5. Vorrichtung zur Beschickung eines Fermenters einer Biogasanlage durch Wärmerückgewinnung aus dem Dünnschlammanteil vergorener Biomasse umfassend:
einen ersten mechanischen Separator (PS1), mit einem ersten Einlass (BE1) für die Biomasse, einen ersten Feststoffauslass (FA1), und einen ersten Dünnschlammauslass (A1),
einen Fermenter (F) mit einem Feststoffeinlass (FE), einen zweiten Dünnschlammeinlass (E2) und einem Gärresteauslass (GA),
einen Wärmetauscher (WT) mit einem ersten Dünnschlammeinlass (E1), einem zweiten Dünnschlammauslass (A2), einen dritten Dünnschlammeinlass (E3) und einen vierten Dünnschlammauslass (A4),
einen zweiten mechanischen Separator (PS2), mit einem zweiten Eingang (BE2), einem dritten Dünnschlammauslass (A3) und einem zweiten Feststoffauslass (FA2) und ein Gärrestelager (GL) wobei der erste mechanische Separator (PS1) über eine erste Leitung (L1) mit der Vorgrube (VG), sein Feststoffauslass (FA1) mit dem Feststoffeinlass (FE) des Fermenters (F) und sein Dünnschlammauslass (A1) mit dem Dünnschlammeinlass (E1) des Wärmetauschers (WT) verbunden ist, wobei der zweite Dünnschlammauslass (A2) des Wärmetauschers mit dem zweiten Dünnschlammeinlass (E2) des Fermenters (FE) verbunden ist,
wobei der Gärresteauslass (GA) des Fermenters (FE) auf den zweiten Eingang (BE2) des Separator (PS2) geführt ist und
wobei der dritte Dünnschlammauslass (A3) des zweiten Separators (PS2) mit dem dritten Dünnschlammeinlass (E3) des Wärmetauschers und der vierte Dünnschlammauslass (A4) des Wärmetauschers (WT) mit dem Gärrestelager (GA) verbunden ist.

6. Vorrichtung nach Anspruch 5, wobei der zweite Feststoffausgang (FA2) des zweiten Separators (PS2) auf den Fermenter zurückgeführt ist.

## Claims

1. Device for feeding a fermenter of a biogas plant by volume reduction of biomass stored in a tank (VG) comprising
a mechanical separator (PS) with an inlet (BE) for the biomass, a solid matter outlet (FA), and a thin sludge outlet (DuA),
a fermenter (F) with a solid matter inlet (FE), a thick sludge inlet (DiE) and a digestate outlet (GA),
an evaporation system (VA) with a thin sludge inlet (DuE), a thick sludge outlet (DiA), a condensate outlet (KA), and an outlet (AS) for ammonium sulfate solution produced,
wherein the mechanical separator (PS) is connected via a first line (L1) with the tank (VG), its solid matter outlet (FA) is connected with the solid matter inlet (FE) of the fermenter (F), and its thin sludge outlet (DuA) is connected with the thin sludge inlet (DuE) of the evaporation system (VA), and wherein the thick sludge outlet (DiA) of the evaporation system (VA) is lead to the thick sludge inlet (DiE) of the fermenter (F).

2. Device according to claim 1, wherein the digestate outlet (GA) of the fermenter (F) is lead via a second line (L2) to the inlet (BE) for the biomass, and via a line L5 to a first digestate outlet (GA1),
wherein the thick sludge outlet (DiA) of the evaporation system (VA) is lead via a fourth line (L4) to a second digestate outlet (GA2), and wherein the first up to the fifth line (L1 to L5) are each provided with a first up to fifth slide (S1 to S5).

3. Device for feeding a fermenter of a biogas system by volume reduction of a biomass stored in a tank (VG), wherein the biomass is pumped from the tank (VG) into a mechanical separator (PS), and is separated there into solid matter and thin sludge, wherein the thin sludge is evaporated to thick sludge in an evaporation system (VA), and wherein the solid matter as well as the thick sludge are lead into a fermenter (F).

4. Device according to claim 3, wherein the digestate fermented in the fermenter (F) is lead back to the separator (PS), and are discharged from the evaporation system (VA) via a second digestate outlet (GA2) after at least one pass.

5. Device for feeding a fermenter of a biogas system by heat recovery from the thin sludge portion of fermented biomass, comprising :
a first mechanical separator (PS1) with a first inlet (BE1) for the biomass, a first solid matter outlet (FA1), and a first thin sludge outlet (A1),
a fermenter (F) with a solid matter inlet (FE), a second thin sludge inlet (E2) and a digestate outlet (GA),
a heat exchanger (WT) with a first thin sludge inlet (E1), a second thin sludge outlet (A2), a third thin sludge inlet (E3), and a fourth thin sludge outlet (A4),
a second mechanical separator (PS2) with a second inlet (BE2),
a third thin sludge outlet (A3) and a second solid matter outlet (FA2) and a digestate store (GL), wherein the first mechanical separator (PS1) is connected via a first line (L1) with the tank (VG), its solid matter outlet (FA1) is connected with the solid matter inlet (FE) of the fermenter (F), and its thin sludge outlet (A1) is connected with the thin sludge inlet (E1) of the heat exchanger (WT), wherein the second thin sludge outlet (A2) of the heat exchanger is connected with the second thin sludge inlet (E2) of the fermenter (FE),
wherein the digestate outlet (GA) of the fermenter (FE) is lead to the second inlet (BE2) of the separator (PS2), and
wherein the third thin sludge outlet (A3) of the second separator (PS2) is connected with the third thin sludge inlet (E3) of the heat exchanger, and the fourth thin sludge outlet (A4) of the heat exchanger (WT) is connected with the digestate store (GL).

6. Device according to claim 5, wherein the second solid matter outlet (FA2) of the second separator (PS2) is lead back to the fermenter.

## Revendications

1. Dispositif pour l'alimentation d'un fermenteur d'une installation de biogaz par réduction de volume de la biomasse stockée dans une préfosse (VG), comportant
une sortie de matière solide (FA), et une sortie de boues fines (DuA), un fermenteur (F) avec une entrée de matière solide (FE), une entrée de boues épaisses (DiE), et une sortie de digestats (GA),
une installation d'évaporation (VA) avec une entrée de boues fines (DuE), une sortie de boues épaisses (DiA), une sortie de condensat (KA), et une sortie (AS) pour la solution de sulfate d'ammonium produite,
dans lequel le séparateur mécanique (PS) est raccordé via une première ligne (L1) à la préfosse (VG), sa sortie de matière solide (FA) est raccordée à l'entrée de matière solide (FE) du fermenteur (F), et sa sortie de boues fines (DuA) est raccordée à l'entrée de boues fines (DuE) de l'installation d'évaporation (VA), et dans lequel la sortie de boues épaisses (DiA) de l'installation d'évaporation (VA) est dirigée à l'entrée de boues épaisses (DiE) du fermenteur (F).

2. Dispositif selon la revendication 1, dans lequel la sortie de digestats (GA) du fermenteur (F) est dirigée via une seconde ligne (L2) à l'entrée (BE) pour la biomasse et via une ligne L5 à une première sortie de digestats (GA1),
dans lequel la sortie de boues épaisses (DiA) de l'installation d'évaporation (VA) est dirigée via une quatrième ligne (L4) à une seconde sortie de digestats (GA2), et dans lequel la première jusqu'à la cinquième ligne (L1 à L5) chacune sont dotées d'une première à cinquième vanne (S1 à S5).

3. Dispositif pour l'alimentation d'un fermenteur d'une installation de biogaz par réduction de volume de la biomasse stockée dans une préfosse (VG), dans lequel la biomasse est pompée à partir de la préfosse (VG) dans un séparateur mécanique (PS), et est séparé là-bas en matière solide et boues fines, dans lequel les boues fines sont évaporées en boues épaisses dans une installation d'évaporation (VA), et dans lequel la matière solide ainsi que les boues épaisses sont dirigées dans un fermenteur (F).

4. Dispositif selon la revendication 3, dans lequel les digestats fermentés dans le fermenteur (F) sont retournés au séparateur (PS), et sont évacués de l'installation d'évaporation (VA) via une seconde sortie de digestats (GA2) après au moins un passage.

5. Dispositif pour l'alimentation d'un fermenteur d'une installation de biogaz par récupération de chaleur à partir de la partie de boues fines de la biomasse fermentée, comportant :
un premier séparateur mécanique (PS1) avec une première entrée (BE1) pour la biomasse, une première sortie de matière solide (FA1), et une première sortie de boues fines (A1),
un fermenteur (F) avec une entrée de matière solide (FE), une seconde entrée de boues fines (E2), et une sortie de digestats (GA),
un échangeur de chaleur (WT) avec une première entrée de boues fines (E1), une seconde sortie de boues fines (A2), une troisième entrée de boues fines (E3), et une quatrième sortie de boues fines (A4),
un second séparateur mécanique (PS2) avec une seconde entrée (BE2),
une troisième sortie de boues fines (A3) et une seconde sortie de matière solide (FA2), et un stockage de digestats (GL), dans lequel le premier séparateur mécanique (PS1) est raccordé via une première ligne (L1) avec la préfosse (VG), sa sortie de matière solide (FA1) est raccordée à l'entrée de matière solide (FE) du fermenteur (F), et sa sortie de boues fines (A1) est raccordée à l'entrée de boues fines (E1) de l'échangeur de chaleur (WT), dans lequel la seconde sortie de boues fines (A2) de l'échangeur de chaleur est raccordée à la seconde entrée de boues fines (E2) du fermenteur (F),
dans lequel la sortie de digestats (GA) du fermenteur (F) est dirigée à la seconde entrée (BE2) du séparateur (PS2), et
dans lequel la troisième sortie de boues fines (A3) du second séparateur (PS2) est raccordée à la troisième entrée de boues fines (E3) de l'échangeur de chaleur, et la quatrième sortie de boues fines (A4) de l'échangeur de chaleur (WT) est raccordée au stockage des digestats (GA).

6. Dispositif selon la revendication 5, dans lequel la seconde sortie de matière solide (FA2) du second séparateur (PS2) est retournée au fermenteur.
